# EUROPEAN PATENT APPLICATION

(11) **EP 1 174 512 A1**
(43) Date of publication of application: **23.01.2002**
(21) Application number: 00810631.2
(22) Date of filing: 17.07.2000
(51) Int. Cl.: C12N 15/82, A01H 5/10

(54) **Seed composition and method for reducing or preventing the release of genetically manipulated pollen**

(71) Applicant: Stamp, Peter, 8049 Zürich (CH)
(72) Inventor: Stamp, Peter, 8049 Zürich (CH); Feil, Boy, 8600 Dübendorf (CH)
(74) Representative: Liebetanz, Michael, Dipl.-Phys.

(57) **Abstract**

The present invention relates to a seed composition and a method for reducing and , if desired, for preventing the release of pollen from genetically modified crops by cultivating GMO plant filed stands in a male sterile version, i.e. without the ability to produce functional pollen.

## Description

The present invention relates to a seed composition and a method for reducing and for preventing the release of pollen from genetically modified (GMO), i.e. transgenic crops by cultivating GMO plants on farmer's fields in a male sterile version, i.e. without the ability to produce pollen or functional pollen.

Crops which are grown for grain production usually release a high number of pollen. Cross-pollinating crops such as maize (*Zea mays L.*) can produce several million pollen grains per plant. Under favourable conditions, the pollen grains remain viable for up to 24 hours and can be transported on the airflow over longer distances. Pollen dispersal away from the vicinity of the crops can also take place by carriage on insects such as bees. In this way, pollen can be transported several kilometers from the crop plot.

Pollen dispersal has long been of interest in seed production as the potential exists for contamination of one crop variety with the pollen of another. Interest in pollen dispersal has recently been renewed with the advent of GMO plants for several reasons:
- Pollen from fields planted with GMO plants may fertilise plants in fields planted with wildtype plants, which results in grains containing the transgene even though no effects are visible. Such products may create problems when thresholds are set for the content of transgenes in seeds and grains. For example, transgenes are not tolerated in organically grown goods.
- Negative impacts of GMO pollen on non-target organisms. For example, it has been reported that monarch butterfly larvae fed milkweed leaves artificially coated with pollen from Bt maize ate less, grew slower, and suffered a higher death rate than larvae that consumed milkweed leaves free of maize pollen (Losey et. al. 1999).
- GMO pollen in honey. There is evidence that even maize pollen is collected by honey bees in notable amounts (Hodges 1984)
- Transfer of transgenes to bacteria in the intestines of bees (H.H. Kaatz, University Jena, and S. Wölfl, Hans Knoll Institut für Naturstoff-Forschung, Jena: RP-online Wissenschaft 2000).

So far no method has been described to reduce or even prevent the release of GMO pollen from crop stands. The present invention now provides a new seed composition as well as a method which enables farmers and researchers to work with GMO crops without the undesired spread of GMO pollen over crops growing on adjacent fields by above described ways and reasons.

The present invention overcomes or minimises the above described problems by sowing male sterile GMO seeds in a mixture with male fertile, wildtype seeds or male fertile GMO seeds, so that the total crop stand will have a normal grain set and yield with reduced release or without the release of GMO pollen.

### Male sterility

Plants can be rendered male sterile by mechanical, chemical or genetic processes. Mechanically and chemically induction of male sterility is very time consuming or often does not fulfil up to now the criteria of low toxicity or reliability, respectively.

Nuclear male sterility can be obtained by plant transformation techniques but crossing a nuclear male sterile mother leads to a segregation of male sterile and fertile offspring.

Cytoplasmic male sterility (cms) is based on an interaction between mitochondrial genes and nuclear genes which leads to dysfunctional pollen. Certain nuclear genes can overcome this effect when they are introduced by crossing into cms-plants, restoring the male fertility. Such genetic systems have been introduced in many crop species for the production of cheap hybrid seeds. When cms-maize plants are pollinated by fertile maize plants they often produce higher yields than their isogenic male fertile counterparts (Stamp et al., in press).

### Cultivation of mixtures from GMO male sterile plants and fertile plants in crop stands

In dependence of the target trait male sterile GMO plants from species with a sufficiently high pollen shed can be cultivated in random mixtures or row-wise with wild type (wildtype), male fertile plants or with GMO, male fertile plants. The latter can be isogenic or non-isogenic to the above-mentioned GMO plants without reducing the yield potential when pollen release and demand of the mixture is synchronised. It is essential that a male sterile system is chosen which reliably prevents the development of functional pollen under field conditions.

It is therefore the object of the present invention to reduce or prevent the release of GMO pollen from crop stands by
a) choosing a male sterile version of a GMO crop plant;
b) choosing isogenic or non-isogenic pollen donor plants which can be wildtype, male fertile for the purpose of preventing the GMO pollen release or GMO, male fertile for the reduction of GMO pollen release;
c) growing the GMO male sterile plants and the pollen donor plants together, in random mixtures or row-wise, thereby allowing for pollination of the GMO male sterile plants by the plants of the pollen donor.

The novel method of the present invention reduces or prevents the release of GMO pollen from crop stands or prevents or reduces the release from functional pollen from crop stands. Thus undesirable impacts like the outcrossing to fields with wildtype of the same species, a direct unwanted effect of the GMO pollen on other organisms and the content of GMO pollen in natural products like honey are minimised or avoided. This is achieved by a mixture between a male sterile version of a GMO plant, incapable or less capable of producing pollen or functional pollen, and male fertile, wildtype plants or male fertile GMO plants which will pollinate all plants in this mixture. Such mixtures can be cultivated for crop species which produce a sufficient surplus of pollen for a high proportion of plants with the GMO trait within the said mixture. Such a crop stand will have a normal grain set and yield with reduced or without the release of GMO pollen.

### Definitions

The following terms are defined:
- Farmer's field:
   This is a community of plants which is cultivated on a field for agricultural products
- Cross-pollination:
   the pollination of the ovules of a plant by another plant which is non-isogenic to it, see non-isogenic
- GMO (= genetically manipulated organism):
   a state where an organism or a part of it has been genetically modified by introducing DNA fragments by using biotechnological methods
- Grains:
   Seeds, achenes and caryopses produced as agriculture commodities
- Male sterile:
   a plant without production of pollen or without production of functional pollen because of mechanical castration or the chemical and genetic induction of sterility in general
- Non-isogenic:
   a state of genetic dissimilarity between individuals when their nuclear genomes possess less than 87% statistical similarity
- Seeds:
   Seeds, achenes and caryopses for reproduction purposes
- Wildtype:
   A state where an organism has not been genetically manipulated

### References

The following references are cited:
Emberlin J. 1999: A report on the dispersal of pollen. http://www soilassociation
org/802566fa00518b30.nsf165e558bf26622f9f802567240041836/f7831ab 0223a763e80256728005af3bd Open Document&Highlight=2, emberlin Losey J.E., L.S. Raynor, and P.C. Lyons 1999: Transgenic pollen harms monarch larvae. Nature 399, 214.
Hodges D. 1984: The pollen loads of the honey bee. International Bee Research Association, London.
RP-online Wissenschaft 2000: Gentransfer von Pflanze auf Bakterium. 24.05.00.
Stamp P., S. Chowchong, M. Menzi, U. Weingartner, and O. Käser 2000: Increase in the yield of cytoplasmatic male sterile maize revisited. Crop Sci. (in press)

Crop species grown for the purpose of grain production such as cereals, rape seed (*Brassica napus* L.) and sunflower (*Helianthus annuus L.*) are the major staple food for mankind, important sources of energy-rich fodder, and the basis for plant derived oils. For many crops of international importance such as maize and rape seed GMO traits have been introduced into commercial varieties which, for example, can induce tolerance to pests like the European corn borer.

Crops like maize produce a high surplus of pollen which can be air-borne in a viable state to considerable distances from the plant direction (Table 1). This example demonstrates that a high number of functional pollen occurs even at long distances from a single plant. This can create major problems when GMO pollen per se or the pollination of wildtype plants with GMO pollen is undesirable for reasons described above.

### Example

It has been demonstrated in field tests that male sterile versions of maize varieties, pollinated by fertile versions of the same variety, often yield even higher than their fertile versions (Table 2, derived from Stamp et al., in press). For crops with a sufficient pollen production, for example maize and rape seed, in isogenic and non-isogenic mixtures with less than 20 % fertile plants a reliable pollination of all plants is achieved. For this reason an undesirable release of GMO pollen can be averted or minimised when GMO crops are cultivated in a male sterile version, pollinated in a mixture by wildtype male fertile versions or by male fertile GMO versios. The composition of the seeds for reducing or preventing the release of pollen from GMO crops comprises a mixture of 5% to 50% seeds for male fertile varieties which are isogenic or non-isogenic to the GMO variety and 50% to 95% seeds for male sterile female GMO plants. Preferably, the mixture comprises 20% for male fertile varieties and 80% for male sterile GMO varieties.

This invention can be applied for all crops which produce a sufficient surplus of pollen for the air-borne or insect-borne pollination of neighbouring plants. In the case of target traits which do exclude the presence of a wildtype plant like in the case of herbicide tolerance, the amount of GMO pollen which is released from farmer's fields can be largely reduced by cultivating mixtures of male sterile GMO plants with male fertile GMO plants as described above. In the case of GMO traits which should not be too much diluted by male fertile wildtype pollinators mixtures of male fertile GMO pollinators and wildtype pollinators can be chosen.

The present invention allows a high grain biomass yield of the whole plant stand without release of GMO pollen or with a reduced release of GMO pollen.

**Table 1:**

| Estimated relative concentration and absolute number of pollen in relation to the distance from the pollen donor (Emberlin 1999) | | |
|---|---|---|
| Distance from the pollen donor (downwind) | Pollen concentration in the air (100 = concentration in 1 m distance from the plant) | Absolute pollen number from a single plant (basis 25 million pollen grains released from one plant) |
| 60 m | 2% | 500 000 |
| 200 m | 1,1 % | 275 000 |
| 500 m | 0.5 - 0.75 % | 125 000 - 187 500 |

**Table 2:**

| Grain biomass yield (g x m⁻²) of the male sterile cms Swiss hybrids Corso and Silex averaged over two years. Changes (%) in relation to the fertile version are presented in parenthesis. | | |
|---|---|---|
| | Density (m⁻²) | Yield |
| Corso | 9 | 961 ( 9.7) |
| Corso | 12 | 1029 (19.3) |
| Silex | 9 | 934 (3.9) |
| Silex | 12 | 942 (3.1) |

## Claims

1. A seed composition for reducing and preventing the release of pollen from genetically manipulated (GMO) crops or for reducing or preventing the release of functional GMO pollen from said crops comprising a mixture of 5% to 50% seeds for male fertile pollinator crops and 50% to 95% seeds for male sterile GMO crops, isogenic or non-isogenic to said male fertile pollinator crops.

2. A seed composition according to claim 1, comprising a mixture of 20% seeds for male fertile pollinator crops and 80% seeds for male sterile GMO crops.

3. A method of GMO crops which reduces of prevents the release of GMO pollen or reduces or prevents the release of functional GMO pollen without loss of grain yield comprising the steps of:
i. selecting a seed mixture of one or more male sterile versions of a GMO crop variety and one or more male fertile isogenic or non-isogenic varieties which are not genetically manipulated; and
ii. sowing said seeds of both types thereby allowing for pollination of GMO male sterile plants by wildtype male fertile plants.

4. A method according to claim 3, comprising the use of a mixture of 5% to 50% seeds for male fertile pollinator crops and 50% to 95% seeds for male sterile GMO crops.

5. A method according to claims 3 and 4, comprising the use of a mixture of 20% seeds for male fertile pollinators crops and 80% seeds for male sterile female GMO crops.

6. A method according to claims 3 to 5 wherein said male fertile pollinators are wildtype.

7. A method according to claims 3 to 6 wherein said male fertile pollinators are GMO.

8. A method according to claim 3, comprising the use of a mixture of 5% to 25% of seeds for male fertile wildtype pollinator crops, 5% to 25% of seeds of male fertile GMO crops and 50% to 90% seeds of male sterile GMO crops.

9. A method according to claim 8, comprising the use of 10% seeds for male fertile wildtype pollinator crops, 10% seeds for male fertile GMO crops and 80% seeds of male sterile female GMO crops.

10. A method according to claims 3 to 9 wherein a random mixture of GMO and wildtype seeds is sown.

11. A method according to claim 3 to 9, wherein the GMO and wildtype seeds are sown in separate rows.

12. A method for planting a field according to one of claims 3 to 11 comprising the steps of planting within a field:
i. male sterile GMO seeds to produce male sterile female plants that do not release pollen or do not release functional pollen;
ii. the seeds of one or more male fertile varieties, isogenic or non-isogenic to the GMO variety, for pollination of said GMO variety;
iii. permitting a high grain biomass yield of both varieties without the release or with reduced release of GMO pollen.
